(19) Europäisches Patentamt
European Patent Office
Office européen des brevets

(11) Publication number: **0 084 677**
**B1**

# (12) EUROPEAN PATENT SPECIFICATION

(45) Date of publication of patent specification: **27.03.85**

(51) Int. Cl.[4]: **C 07 C 69/96**, C 07 C 68/02, A 61 K 31/265

(21) Application number: **82112026.8**

(22) Date of filing: **27.12.82**

(54) **New compound having mucoregulating and fluidifying activity, process for its production and relative pharmaceutical compositions.**

(30) Priority: **21.01.82 IT 1921482**

(43) Date of publication of application:
**03.08.83 Bulletin 83/31**

(45) Publication of the grant of the patent:
**27.03.85 Bulletin 85/13**

(84) Designated Contracting States:
**AT BE CH DE FR GB IT LI LU NL SE**

(56) References cited:

**None**

(73) Proprietor: **AUSONIA FARMACEUTICI S.r.l.**
**Via Laurentina Km. 24730**
**I-00040 Pomezia (Rome) (IT)**

(72) Inventor: **de Vincentiis, Leonardo**
**Via Isonzo 42**
**Roma (IT)**

(74) Representative: **Bianchetti, Giuseppe**
**Studio Consulenza Brevettuale Via Senato 24**
**I-20121 Milan (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

The present invention relates to 5-[(2-methoxy-phenoxy)carbonyloxy]-α,α,4-trimethyl-3-cyclohexen-1-methanol, having formula (I)

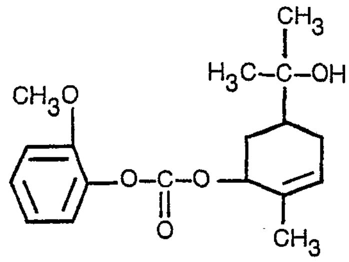

(I)

The therapeutic properties of 2-methyl-5-(1-hydroxy-1-methyl-ethyl)-2-cyclohexen-1-ol, or sobrerol (II)

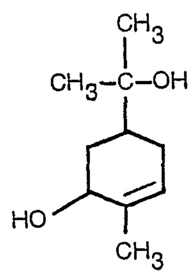

(II)

are known since many years. It has been used for the treatment of respiratory diseases (V. Dalla Valle — Bolle Chim. Farm., 109, (1970)), and more exactly as fluidifying agent with bronchosecretagogue and mucosecretolytic activity in cases of acute and chronic bronchitis with hypersecretive and obstructive component of the respiratory routes (R. Scuri et al., il Farmaco ed. Pr. 36, 1 (1980)).

On the other side, the expectorant properties of 2-methoxyphenol, or guaiacol, are well known. It has now unexpectedly been found that the mixed carbonate of guaiacole and sobrerole, that is compound having formula (I), exhibits bronchosecretogogue and fluidifying properties markedly higher than that both of guaiacol and of sobrerol, with a mucosecretolytic activity comparable to that of the two compounds herewith referred to.

Thus, another object of the invention is provided by pharmaceutical compositions endowed with mucoregulating and fluidifying activities, containing as active principle compound (I).

Finally, the invention relates to a process for the preparation of 5-[(2-methoxy-phenoxy)carbonyloxy]-α,α,4-trimethyl-3-cyclohexen-1-methanol, characterized by reacting guaiacol chlorocarbonate (III) with sobrerol (II), according to the following scheme:

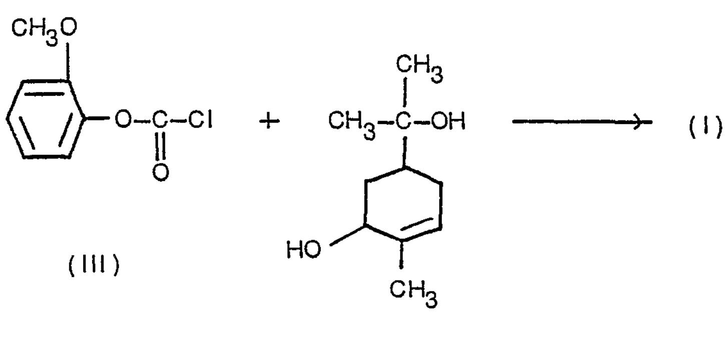

(III) + (II) → (I)

The reaction is carried out in aprotic solvents, for example in ether such as diethylether, tetrahydrofuran, dioxane, and in presence of acidity acceptors. As such, tertiary bases are preferably used, such as pyridine or triethylamine. One can operate at temperatures ranging from 5 to 100°C, preferably from 20 and 80°C.

The following example further illustrates, without limiting it, the process according to the invention.

Example

a) 124 Grams of guaiacol are dissolved in 500 ml of a 20% phosgene solution in toluene, keeping the temperature within 5°C; 130 g of N,N-dimethylaniline, diluted in 300 ml of benzene, are added, keeping the temperature below 15°C. When the addition is over the solution is kept under stirring at room temperature for 30 minutes. The reaction mixture is then washed with water and then with diluted $H_2SO_4$. The separated organic phase is dried on anhydrous sodium sulphate, filtered and concentrated under reduced pressure to dryness.

The crude guaiacol chlorocarbonate is distilled at the mechanical pump; 150 g of pure product are then collected at the temperature of 50°C/0.15 mm Hg.

b) To a stirred solution of 70 g of sobrerol (dl-trans form) in 500 ml of tetrahydrofuran, 80 g of guaiacol chlorocarbonate dissolved in 200 ml of tetrahydrofuran are first added and then, in portions, 40 g of pyridine. The temperature raises to about 60°C, while a precipitate is formed. Stirring is continued for about two hours, the solvent is then evaporated under reduced pressure, the residue is treated with 500 g of water and ice, the oil is extracted with ether. The ether phase is washed with 2% acqueous HCl, then with water, thereafter with saturated soluzione of $NaHCO_3$. By solvent's evaporation to dryness a semi solid product is obtained which is re-crystallised from diisopropylether (the solution is cooled to 0°C). In such a way 94.4 g of compound (I) are obtained, m.p. 62°C, almost insoluble in water, soluble in alcohols, acetone, diethylether, chloroform. The structure of compound (I) is confirmed by analytical and spectroscopical data.

*Elemental analysis:* for $C_{18}H_{24}O_5$ (M.W. = 320.38)
calcd. % C = 67.48; H = 7.55
found % C = 67.62; H = 7.59.

*I.R. Spectrum* (nujol mull; absorption bands values are expressed in $cm^{-1}$):

| | |
|---|---|
| stretch O—H | 3550—3450 |
| stretch C—H aromatics | 3080 |
| stretch C—H alifatics | 2980—2840 |
| stretch C=O carbonate | 1770 |
| stretch C=C | 1620 |

*$H^1$ NMR Spectrum* (recorded in $CDCl_3$, internal reference TMS; values of chemical shifts are expressed in δ):

1.2 (d, 6H, 2($CH_3$));
1.8 (d, 3H, =C—$CH_3$);
1.4—2.3 (m, 6H, <u>$CH_2$</u>—<u>CH</u>—<u>$CH_2$</u>, OH mobile);
3.8 (s, 3H, $OCH_3$);
5.3 (m, 1H, CH—O—CO);
5.8 (m, 1H, =<u>CH</u>—$CH_2$);
6.8—7.3 (m, 4H aromatics).

The bio-pharmacological characteristics of compound (I) — which will be hereinafter defined, for the sake of brevity, with the abbreviation AFP 736 — have been determined as hereinunder described.

*Toxicity*

The acute toxicity has been determined in Swiss mouse and in Wistar rat for different administration routes, according to the method of Litchfield J. T. and Wilcoxon [J. Pharm. Exp. Therap. *96,* 99—113 (1949)]. The results obtained are reported in the following Table I; from the same it can be pointed out how AFP 736 is endowed with a very low acute toxicity.

TABLE I

Acute toxicity of AFP 736

| Species | Administration route | $LD_{50}$ – (mg/kg) |
|---|---|---|
| mouse | os<br>i.p. | 3100<br>1750 |
| rat | os<br>i.p. | >4000<br>2340 |

*Bronchosecretogogue activity*

The bronchosecretogogue activity has been determined in male Wistar rat by per os administration according to the method of Mawatari [Mawatari H., Kagoshima Daigaku, Igaku-Zasshi, *27,*, 561 (1976)], comparing AFP 736 with carboxymethylcysteine, sobrerol and guaiphenesin at the doses hereinafter stated. The results are reported in the enclosed Table 2.

TABLE 2

Bronchosecretogogue activity (Mawatari)

| Compound | Administered Dose mg/kg/os | No. of animals | % Increase of FlNa(*) in comparison with controls |
|---|---|---|---|
| AFP 736 | 500 | 10 | 79.3 |
| Carboxy-methyl-cysteine | 500 | 10 | 39.7 |
| Soberol | 500 | 10 | 33.4 |
| Guaiphe-nesine | 500 | 10 | 12.6 |

(*) FlNa = Sodium fluoresceine.

From the resulting values a more marked activity of AFP 736 in the test under exam clearly turns out in comparison with reference drugs used at equiponderant doses.

*Mucosecretolytic activity*

The mucrosecretolytic activity has been studied in the male rabbit according to the technique of Giraldez, Grass and Bruseghini (Abstr. Comp. Int. Pharm. N. 1086—Paris 1978), by administration by the oral route, comparing AFP 736 with carboxymethylcysteine and sobrerol at the doses hereinafter stated. The results are reported in the Table 3.

TABLE 3

Mucosecretolytic activity (tecnique of Giraldez et al.)

| Compound | Dose mg/kg/os | No. animals | % Increase of secretion in comparison with controls |
|---|---|---|---|
| AFP 736 | 500 | 10 | 45.5 |
| Carboxy-methyl-cysteine | 500 | 10 | 40.2 |
| Sobrerol | 500 | 10 | 36.8 |

From the comparison of the results obtained by the test under exam the the mucosecretolytic activity of AFT 736 results comparable to that of carboxymethylcysteine used at equiponderant doses and remarkably higher than that of sobrerol, always at equiponderant doses.

*Fluidifying activity "in vivo"*

The fluidifying effect has been determined with the microviscosimeter on the tracheo-bronchial mucus from rabbit after administration by oral route, comparing AFP 736 with sobrerol and guaiacol, at the doses hereinafter stated. The results are reported in the enclosed Table 4.

TABLE 4

Fluidifying activity "in vivo" in the rabbit

| Compound | Dose mg/kg/os | No. animals | % Reduction of mucus viscosity |
|---|---|---|---|
| AFP 736 | 500 | 5 | 32.2 |
| Sobrerol | 500 | 5 | 15.6 |
| Guaiacol | 500 | 5 | 4.1 |

As it turns out from the values obtained the fluidifying activity of AFP 736 proves to be remarkably enhanced in comparison with reference drugs employed at equiponderant doses. Compound (I) is therefore fit as an elective drug with mucroregulating and fluidifying activity, in the therapy of acute and chronic diseases of the respiratory apparatus, particularly of bronchitis, laryngo tracheitis, cough accommpanied by catarrh. The present invention refers also to all the industrially applicable aspects connected with the use of AFP 736 as mucoregulating and fluidifying agent. An essential aspect of the invention is therefore provided by pharmaceutical compositions containing predetermined amounts of AFP 736. Such a compound can be administered, particularly, by the oral route, as capsules, extemporary suspension, monodose sachets (in a granular state); or by aerosol; or as suppositories; or, finally, by parenteral route (vials for injection).

Of course, the single pharmaceutical forms will contain the active principle together with vehicles, excipients, flavouring agents etc. of conventional use in pharmaceutical technique.

**Claims for the Contracting States: BE CH DE FR GB IT LI LU NL SE**

1. As novel compounds, 5-[(2-methoxy-phenoxy)carbonyloxy]-α,α,4-trimethyl-3-cyclohexen-1-methanol, having formula (I)

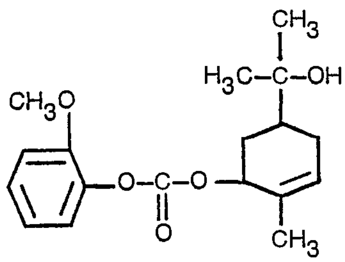

(I)

2. Pharmaceutical compositions endowed with bronchosecretogogue, mucosecretolytic and anti-cough activity, characterized by containing as active principle the compound of claim 1.

3. Process for the preparation of 5-[(2-methoxy-phenoxy)carbonyloxy]-α,α,4-trimethyl-3-cyclohexen-1-methanol, having formula (I), characterized by reacting guaiacole chlorocarbonate (III) with sobrerol (II), according to the following scheme:

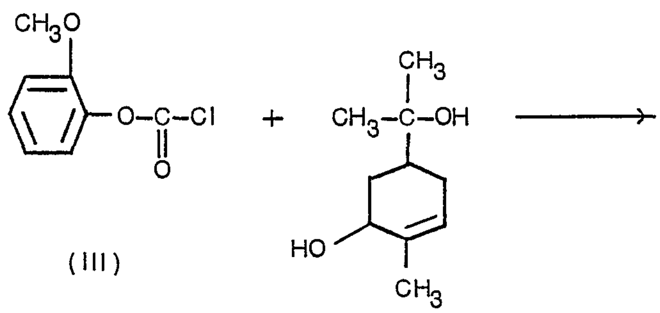

(III) (II)

4. Process according to claim 3, characterized by operating in inert solvents, such as diethylether, tetrahydrofuran or dioxan.

5. Process according to claims 3—4, characterized by operating in the presence of acidity acceptors, such as triethylamine or pyridine.

6. Process according to claims 3—5, characterized by operating at temperatures ranging from about 5 to 100°C, preferably from 20 to 80°C.

**Claims for the Contracting State: AT**

1. Process for the preparation of 5-[(2-methoxy-phenoxy)carbonyloxy]-α,α,4-trimethyl-3-cyclohexen-1-methanol, having formula (I)

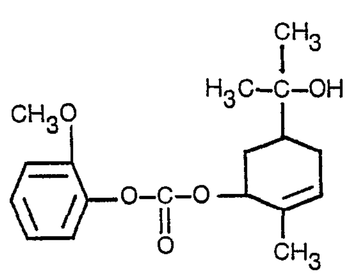

(I)

characterized by reacting guaiacole with chlorocarbonate (III) with sobrerol (II), according to the following scheme:

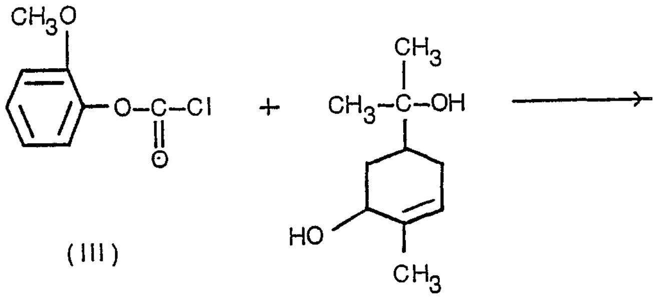

(I)

(III)

(II)

2. Process according to claim 1, characterized by operating in inert solvents, such as diethylether, tetrahydrofuran or dioxan.

3. Process according to claim 1—2, characterized by operating in the presence of acidity acceptors, such as triethylamine or pyridine.

4. Process according to claims 1—3, characterized by operating at temperatures ranging from about 5 to 100°C, preferably from 20 to 80°C.

**Patentansprüche für die Vertragsstaaten: BE CH DE FR GB IT LI LU NL SE**

1. Als eine neue Verbindung 5-[(2-Methoxy-phenoxy)-carbonyloxy]-α,α,4-trimethyl-3-cyclohexen-1-methanol der Formel (I)

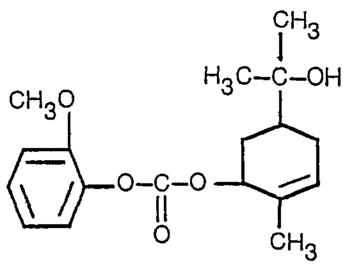

(I)

2. Pharmazeutische Zusammensetzungen mit bronchosekretogoischer, mucosekretolytischer und Antihusten-Aktivität, dadurch gekennzeichnet, daß sie als aktives Prinzip die Verbindung von Anspruch 1 enthalten.

3. Verfahren zur Herstellung von 5-[(2-Methoxyphenoxy)carbonyloxy]-α,α,4-trimethyl-3-cyclohexen-1-methanol der Formel (I), gekennzeichnet durch Umsetzen von Guaiacol-chlorcarbonat (III) mit Sobrerol (II) gemäß folgendem Schema

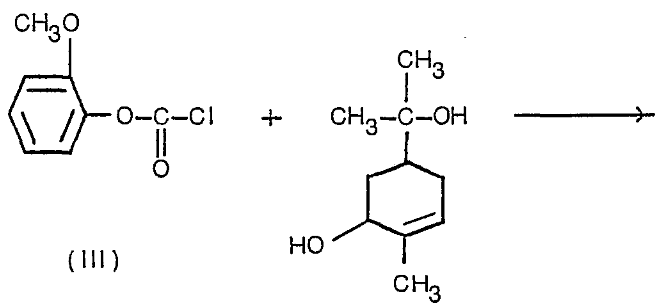

(I)

(III)

(II)

4. Verfahren nach Anspruch 3, gekennzeichnet durch Arbeiten in inerten Lösungsmitteln, wie Diethylether, Tetrahydrofuran oder Dioxan.

5. Verfahren nach den Ansprüchen 3 bis 4, gekennzeichnet durch Arbeiten in Gegenwart von Säureakzeptoren, wie Triethylamin oder Pyridin.

6. Verfahren nach den Ansprüchen 3 bis 5, gekennzeichnet durch Arbeiten bei Temperaturen im Bereich von etwa 5 bis 100°C, bevorzugt von 20 bis 80°C.

**Patentansprüche für den Vertragsstaat: AT**

1. Verfahren zur Herstellung von 5-[(2-Methoxy-phenoxy)carbonyloxy]-α,α,4-trimethyl-3-cyclohexen-1-methanol der Formel (I)

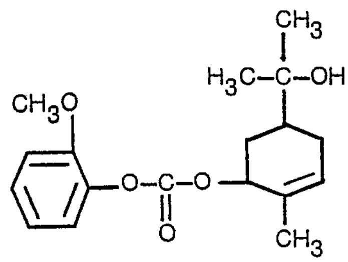

(I)

gekennzeichnet durch Umsetzen von Guaiacol-chlorcarbonat (III) mit Sobrerol (II) gemäß folgendem Schema

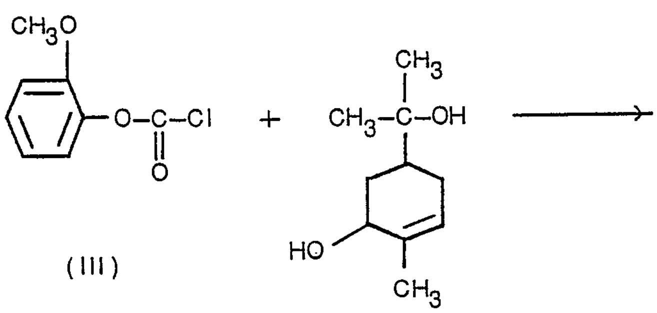

(I)

(III)

(II)

2. Verfahren nach Anspruch 1, gekennzeichnet durch Arbeiten in inerten Lösungsmitteln, wie Diethylether, Tetrahydrofuran oder Dioxan.

3. Verfahren nach Anspruch 1 bis 2, gekennzeichnet durch Arbeiten in Gegenwart von Säureakzeptoren, wie Triethylamin oder Pyridin.

4. Verfahren nach den Ansprüchen 1 bis 3, gekennzeichnet durch Arbeiten bei Temperaturen im Bereich von etwa 5 bis 100°C, bevorzugt von 20 bis 80°C.

**Revendications pour les Etats contractants: BE CH DE FR GB IT LI LU NL SE**

1. Nouveau composé 5-[(2-méthoxy-phénoxy)carbonyloxy]-α,α,4-triméthyl-3-cyclohexen-1-méthanol, ayant la formule (I):

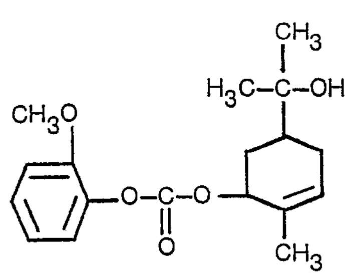

(I)

2. Compositions pharmaceutiques présentant une activité bronchosecrétogogue mucosecrétolytique et antitussive, caractérisées en ce qu'elles contiennent comme principe actif le composé de la revendication 1.

3. Procédé de préparation du 5-[(2-méthoxyphénoxy)carbonyloxy]-α,α,4-triméthyl-3-cyclohexen-1-méthanol, ayant la formule (I), caractérisé en ce qu'on fait réagir le chlorocarbonate de guaiacole (III) avec du sobrerol (II), selon le schéma suivant:

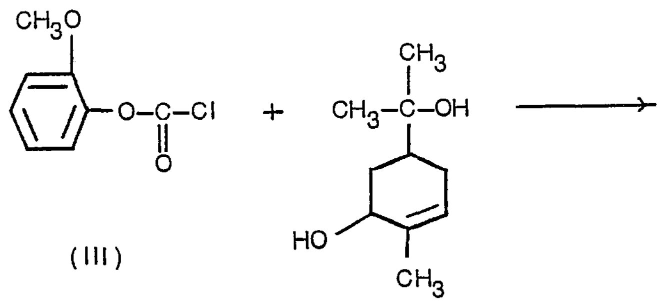

(I)

(III)

(II)

4. Procédé selon la revendication 3, caractérisé en ce qu'on opère dans des solvants inertes, tels que le diéthyléther, le tétrahydrofurane ou le dioxane.

5. Procédé selon les revendications 3 ou 4, caractérisé en ce qu'on opère en présence d'accepteurs d'acidité, tels que la triéthylamine ou la pyridine.

6. Procédé selon les revendications 3 à 5, caractérisé en ce qu'on opère à des températures allant d'environ 5 à 100°C, de préférence de 20 à 80°C.

**Revendications pour l'Etat contractant: AT**

1. Procédé de préparation de 5-[(2-méthoxy-phénoxy)carbonyloxy-α,α,4-triméthyl-3-cyclohexene-1-méthanol, ayant la formule (I)

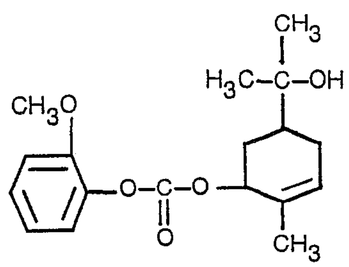

(I)

caractérisé en ce que l'on fait réagir le chlorocarbonate de guaiacole (III) avec du sobrerol (II), selon le schéma suivant:

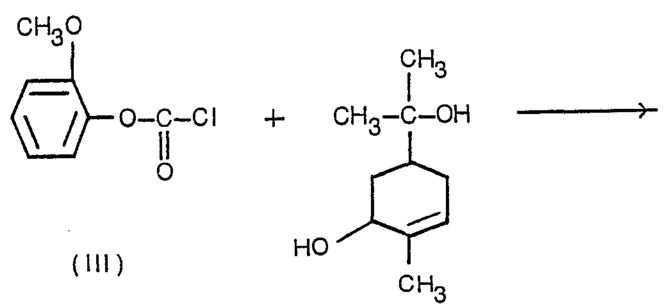

(I)

(III)

(II)

2. Procédé selon la revendication 1, caractérisé en ce que l'on opère dans des solvants inertes, tels que le diéthyléther, le tétrahydrofurane ou le dioxane.

3. Procédé selon les revendications 1 ou 2, caractérisé en ce que l'on opère en présence d'accepteurs d'acidité, tels que la triéthylamine ou la pyridine.

4. Procédé selon l'une des revendications 1 à 3, caractérisé en ce que l'on opère à des températures allant d'environ 5 à 100°C. de préférence de 20 à 80°C.